Europäisches Patentamt

European Patent Office

Office européen des brevets

(19)

(11) Publication number: **0 441 278 A1**

## (12) EUROPEAN PATENT APPLICATION

(21) Application number: 91101424.9

(22) Date of filing: 04.02.91

(51) Int. Cl.⁵: **C08B 37/00, A61K 35/78,**
**A61K 31/715**

(30) Priority: 09.02.90 IT 1932590

(43) Date of publication of application:
**14.08.91 Bulletin 91/33**

(84) Designated Contracting States:
**AT BE CH DE DK ES FR GB GR IT LI LU NL SE**

(71) Applicant: **INDENA S.p.A.**
**Via Ripamonti, 99**
**I-20141 Milano(IT)**

(72) Inventor: **Bombardelli, Ezio**
**Via Ripamonti, 99**
**I-20141 Milano(IT)**
Inventor: **Pozzi, Roberto**
**Via Ripamonti, 99**
**I-20141 Milano(IT)**

(74) Representative: **Bianchetti, Giuseppe**
**Studio Consulenza Brevettuale Via Rossini,**
**8**
**I-20122 Milan(IT)**

(54) **Polysaccharides with immunomodulating properties from astragalus membranaceus and pharmaceutical compositions containing them.**

(57) New glucanes (Astraglucanes) and their mixture with other polysaccharides extracted from the roots and rhizomes of Astragalus membranaceus and other species, endowed with immunomodulating properties, are disclosed. These new products find their application as an aid in radiation antineoplastic therapy, in chemotherapy and in the treatment and prevention of bacterial and viral infections. Said products are active by oral route and after injection and are suitable for incorporation into the most common pharmaceutical formulations.

EP 0 441 278 A1

## POLYSACCHARIDES WITH IMMUNOMODULATING PROPERTIES FROM ASTRAGALUS MEMBRANACEUS AND PHARMACEUTICAL COMPOSITIONS CONTAINING THEM

The present invention refers to polysaccharides with molecular weights between 12000 and 500000 daltons, obtained from plants of the genus Astragalus and characterized by a marked immunomodulating activity which has been found to be useful in the treatment of immunodeficiency syndromes.

The use of polysaccharides of bacterial or vegetal origin in clinical therapy is already known and many investigations are being performed world-wide to identify new molecules endowed with a greater activity or with a more specific mode of action. Among compounds of vegetal origin, mention can be made of those extracted from Echinacea, Sabal serrulata, Eleutherococcus, etc. The polysaccharides extracted from these plants have molecular weights between 25000 and 500000 daltons and have been shown to possess in vivo and in vitro a stimulating effect on granulocytes and macrophages.

Investigations on Astragalus originated from thousands of years of usage of this plant in Chinese medical practice for the prevention and treatment of various viral diseases and cancer. Recently published papers (J. Clin. Lab. Immunol. 25, 119-23, 1988 and J. Clin. Lab. Immunol. 25, 125 125-29, 1988) reported that fractions of Astragalus, not well defined chemically and prepared by means of procedures difficult to reproduce industrially, exert immunostimulating effects which are capable, for example, of reversing the immunodeficiency of T-leukocytes from cancer patients. The fraction defined as F3 on the basis of the preparation sequence, containing 50% of carbohydrates and less than 1% of proteins (the nature of the remaining 49% being unknown) and possessing a molecular weight of 20000-25000 daltons, has also been found to restore the organic defenses impaired by cyclophosphamide treatment. These observations have led to preliminary controlled clinical trials of raw extracts of Astragalus in China. In previous studies (Dissertation Thesis, McLaughlin, 1986), it had been possible to isolate from the roots of Astragalus membranaceus some polysaccharides characterized by molecular weights of about 25000 daltons which consisted of galactose, arabinose and xylose and were endowed with generic immunomodulating properties.

Surprisingly, it has been found that the fractions obtained by using the procedure in the present invention exert greater immunomodulating effects than those exhibited by compounds known from the prior art. The compounds described in the present invention (Astroglucanes A, B and C) are new and characterized by a well defined chemical composition, which can be documented chemically and spectroscopically. Their molecular weights, determined by a combination of Gel Permeation and Laser Light Scattering System, range between 12000 and 500000. Their basic structure consists of sequences of 1,3-beta-glucose with a branched arrangement and repeating olygomeric unit.

To prepare these compounds, the finely ground roots of Astragalus membranaceus are extracted until exhaustion with aliphatic alcohols, preferably with 95% ethanol or with water and subsequently with alcohols starting at a concentration of 20% which may be increased up to 50%, in order to remove in the former case lipophilic substances and low molecular weight compounds and in the latter case polysaccharides with a molecular weight below 30000 daltons. The product is subsequently treated with aqueous alkali, according to known procedures reported in the literature for the isolation of mucopolysaccharide substances (Arzneim. Forsch. 35, 1069-75, 1985), and digested at temperatures between -2°C and 45°C, preferably at room temperature. After a period ranging from 3 to 30 hours, the reaction medium in which the hydrolysis of the proteins and the saponification of various ester groups of the lipids took place is adjusted to a pH between 6 and 7.5 with mineral or organic acids, preferably with acetic acid, and heated to the boiling point for 3 hours. Under these conditions, boiling results in virtually complete denaturation of the proteins, which precipitate on the product, and in solubilization of the polysaccharides. After this treatment, the product is percolated in boiling water and re-extracted under the same conditions until complete exhaustion in the desired polysaccharides, which are then precipitated by treatment with alcohols as described below. The pooled aqueous extracts are concentrated under vacuum to a volume corresponding to 1 to 5 times the weight of the untreated raw vegetal material. The concentrate is clarified by supercentrifugation in order to remove the protein flocculates and the insoluble cellulose materials. The clarified liquid is poured under vigorous agitation into 95% ethanol at a volume ranging from 1 to 4 times the volume of the fluid containing the polysaccharides. Under these conditions an abundant precipitate formed, which after drying is dialyzed through membranes with a cut-off around 10000 in order to remove simple carbohydrates, peptides, amino acids and residual salts. The undesired material mentioned above is discarded and the aqueous phase is concentrated under vacuum and precipitated with an appropriate volume of isopropanol. The isolation of single polysaccharides is carried out by preparative HPLC using exclusion columns (Aquapor) capable of separating products with molecular weights ranging between 10000 and 500000. The isolated products are checked by Gel Permeation and $^{13}$C-NMR.

Alternatively, the alkaline extraction solution may be filtered accurately and diluted with ethanol up to 35% proportion of alcohol in order to directly separate the polysaccharides of high molecular weight described in the present invention. The raw solid material obtained may subsequently be purified by re-solubilization in water at pH 9 and re-precipitation in alcohol in order to remove non-mucopolysaccharide impurities. Alternatively the products described in the present invention may also be isolated by filtering the neutral or alkaline solution through membranes with a suitable cut-off. The main product of the polysaccharide fraction obtained by using the procedure described in the present invention has molecular weight of 456000 and contains as sugars only glucose, determined by GLC with a beta 1,3-glucoside bond determined by NMR.

In vitro and in vivo tests were carried out to evaluate the biological activity of these new polysaccharides on parameters of immune responses dependent on the direct or indirect activation of different immunocyte or accessory cell subpopulations. In vitro, the tests substances, already at a dose as low as 10 mcg/ml, significantly increase the blastization index in mixed lymphocyte cultures and the granulopexis of macrophages or polymorphonucleates derived from human blood. In vivo, already at a dosage of 25 mg/kg given orally or intraperitoneally for three days, these substances exert a significant protecting action against the otherwise fatal infection from Candida albicans in CD1 mice treated with antibiotics aggravating the mycosis (tetracycline). In addition, it has been possible to document in vitro and in vivo the proliferating response of spleen T lymphocytes exposed to various mitogens such as concanavalin A and Escherichia coli lipopolysaccharides in CDF1 mice by adding, after incubation, labelled thimidine. The results mentioned above were obtained by using the pure individual compounds or their mixtures, the latter including also polysaccharide fractions known from prior art, such as those isolated by fractioning the aqueous or hydroalcoholic extract with 40% ethanol and with membranes or gel-filtration.

As far the clinical application of these new therapeutic agents is concerned, surprising results were obtained by treating patients who had undergone surgical excision of tumour masses from the larynx, the colon, the breast and the lung. The benefits achieved in these patients included a fall in relapse rate and a prolongation of survival. These substances were also found to be valuable for the prevention and treatment of infections induced by the most common viruses and by the AIDS virus itself. The polysaccharides described in the present invention can be used alone or in combination with polysaccharides described previously, even though the latter are per se characterized by a lower specific activity. In some cases, the combination of these two products results in synergistic effects which may usefully be applied for less expensive treatments of frequently occurring diseases.

The products described in the present invention may be used in the most common pharmaceutical formulations at dosages ranging from 10 to 100 mg/kg.

The following examples illustrate the methods for the isolation of the products described in the present invention, without representing a limitation in any way.

**EXAMPLE 1: Preparation of Astraglucane A**

10 kg of finely ground roots of Astragalus membranaceus are extracted with 95% ethanol until exhaustion of the substances solubilized by this solvent. The residual vegetal material is re-extracted with boiling water in order to extract the glucide components which will subsequently undergo the fractioning process. The wet residue is treated with 5 volumes of a 1N NaOH solution and left to stand for 12 hours. The alkaline solution is separated and the vegetal material is re-extracted again with 1N NaOH at room temperature. The filtered alkaline solution is neutralized, reduced in volume under vacuum and subsequently diluted with ethanol in a proportion up to 50%. This results in the formation of an abundant precipitate which is filtered and dried under vacuum. This precipitate, which consists of a mixture of high molecular weight polyglucanes, is redissolved in 20 volumes of water and precipitated again in ethanol with a proportion of alcohol up to 35%. The resulting precipitate is a white, solid material which consists predominantly of substances with molecular weight above 200000 daltons. This material is re-dissolved in distilled water. Subsequently, ethanol is added up to a 30% proportion of alcohol. After solubilization in water and filtration, the final material is lyophilized. This procedure yields 350 g of a product having the following physico-chemical characteristics: melting point 270°C (with decomposition), signals of protons on -CH- at 3.5-4.5 ppm at $^1$H-NMR (D$_2$O) testing.

This compound is denominated Astraglucane A and possesses considerable immunomodulating activity.

**EXAMPLE 2:**Preparation of Astraglucane B

10 kg of ground roots of Astragalus membranaceus are extracted with 50 l of boiling water for three times. The aqueous extract is concentrated under vacuum to one third of its original volume and is then diluted with isopropanol up to a 30% proportion of alcohol: the resulting rubber-like precipitate is finely divided in pure isopropanol in order to remove lipide impurities. The residue is re-dissolved in 10 volumes (w/v) of distilled water and the solute is poured under vigorous shaking into an equal volume of isopropanol: an abundant white precipitate forms which is centrifuged and dried. The final solid material consists of a mixture of three glucanes with molecular weights ranging between 125000 and 250000 daltons, the most abundant of which is a glucane with a molecular weight of 213000.

**EXAMPLE 3:**Preparation of the total polysaccharide mixture

10 kg of roots of Astragalus membranaceus are extracted with 100 l of 95% ethanol under reflux for 4 hours. The alcoholic solution is discarded and the vegetal material is extracted with 100 l of 20% aqueous ethanol. The hydroalcoholic extract is concentrated under vacuum to a 10 l volume at a temperature of 50°C. The aqueous solution is decolourized at 50°C with 100 g of activated vegetal charcoal and is then diluted under continuous agitation with 40 l of 95% ethanol. The resulting precipitate is collected by decanting. The supernatant is discarded and the solid material is set apart. The vegetal material obtained by extraction with 20% ethanol, as described above, is treated with 50 l 1N NaOH and left to stand at 14°C for 12 hours. The solution and the vegetal material are neutralized at pH 7 and heated to the boiling point for 2 hours. The vegetal material is filtered at a temperature of about 70°C and the extract, decolourized with 100 g of activated vegetal charcoal, is concentrated to 8 l. After filtering to remove the flocculated material, the concentrate is poured into 10 l of 95% ethanol. The resulting solid precipitate is collected and added to the material obtained above from the neutral hydroalcoholic extraction. The pooled solid materials are dissolved in 5 l of water and the resulting solution is poured under vigorous shaking into 20 l of isopropanol. After drying under vacuum overnight, 420 g of the final product are obtained which consist of a polysaccharide mixture containing substances with molecular weights ranging from 12000 to 500000 daltons.

**EXAMPLE 4:**Preparation of the total polysaccharide fraction

10 kg of finely ground roots of Astragalus membranaceus are treated according to the procedure described in Example 3. After the alkaline treatment of the vegetal material, the extract, instead of being neutralized and heated, is simply diluted with ethanol up to a 20% proportion of alcohol. The precipitate obtained after solubilization and re-precipitation is added to the residues obtained previously and the resulting pooled material suspended in 10 l of water is dialyzed using membranes with cut-off 10000. The non dialyzable phase contains all the desired substances and is lyophilized, to obtain 380 g of a product having marked immunostimulating activity.

**Claims**

1. Polysaccharides of molecular weight from 12000 to 500000 daltons extracted from the roots of plants belonging to the genus Astragalus.

2. Polysaccharides of claim 1, extracted from the roots of Astragalus membranaceus.

3. Polysaccharides of claims 1 and 2, having molecular weight from 125000 to 250000 daltons.

4. Polysaccharides of claims 1 and 2, having molecular weight of 456000 daltons.

5. Polysaccharides of claims 1 and 2, having molecular weight of 213000 daltons.

6. A process for the preparation of the polysaccharides of the preceding claims, in which process finely ground roots of Astragalus membranaceus are subjected to the following steps:
    a) removal of lipophilic substances by extraction with aliphatic alcohols;
    b) removal of polysaccharides of molecular weight below 30000 daltons, by extraction with alcohols containing from about 20 to about 50% water;
    c) digestion with aqueous alkali at temperatures from -2 to +45°C for 3-30 hours;
    d) purification of the polysaccharide fraction by means of one or more of following steps:
    1) precipitation of the proteins by denaturation in acids, followed by extraction of the drug with boiling water and concentration under reduced pressure of the aqueous extracts, which then are subjected to processes of clarification, precipitation, dialysis, preparative HPCL, lyophilization, in appropriate sequence;

2) precipitation of the high molecular weight polysaccharides by dilution with ethanol to a 35% proportion, followed by re-dissolution in water and re-precipitation with alcohol;

3) filtration through membranes with appropriate cut-off.

7. Pharmaceutical compositions containing as the active ingredient the polysaccharides of claims 1-5 in admixture with a suitable carrier.

8. The use of one of the polysaccharides of claims 1-5 for the preparation of a medicament having immunostimulating activity.

**Claim for the Contracting States: ES, GR.**

1. A process for the preparation of polysaccharides of molecular weight from 12000 to 500000 daltons extracted from the roots of plants belonging to the genus Astragalus, in which process finely ground roots of Astragalus membranaceus are subjected to the following steps:

    a) removal of lipophilic substances by extraction with aliphatic alcohols;

    b) removal of polysaccharides of molecular weight below 30000 daltons, by extraction with alcohols containing from about 20 to about 50% water;

    c) digestion with aqueous alkali at temperatures from -2 to $+45°$ C for 3-30 hours;

    d) purification of the polysaccharide fraction by means of one or more of following steps:

    1) precipitation of the proteins by denaturation in acids, followed by extraction of the drug with boiling water and concentration under reduced pressure of the aqueous extracts, which then are subjected to processes of clarification, precipitation, dialysis, preparative HPCL, lyophlization, in appropriate sequence;

    2) precipitation of the high molecular weight polysaccharides by dilution with ethanol to a 35% proportion, followed by re-dissolution in water and re-precipitation with alcohol;

    3) filtration through membranes with appropriate cut-off.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 843 067   (LIU)<br>* Column 1, lines 44-57,62-67; column 2, lines 35-41; column 2, line 63 - column 3, line 52; claims 3-6 * | 1,2,7,8 | C 08 B 37/00<br>A 61 K 35/78<br>A 61 K 31/715 |
| Y | | 6 | |
| Y | EP-A-0 030 444   (KITASATO KENKYUSHO)<br>* Page 3, lines 10-21; page 18, line 34 - page 19, line 34; claims 1-9 * | 6 | |
| D,X | DISSERTATION ABSTRACTS INTERNATIONAL, vol. 47, no. 7, January 1987, page 2891, order no. DA8625417; M.A. McLAUGHLIN: "Identification of immunostimulants derived from the plant astragalus membranaceaus"<br>* Abstract * | 1,2 | |
| A | CHEMICAL ABSTRACTS, vol. 110, no. 1, 2nd January 1989, page 38, abstract no. 463t, Columbus, Ohio, US; X. MAO et al.: "Effect of Hedysari and Astragli polysaccharides on humoral immune function in mice",<br>& ZHONGGUO MIANYIXUE ZAZHI 1988, 4(3), 158-61<br>* Abstract * | 1 | |

TECHNICAL FIELDS
SEARCHED (Int. Cl.5)

C 08 B
A 61 K

The present search report has been drawn up for all claims

| Place of search | Date of completion of search | Examiner |
|---|---|---|
| The Hague | 22 May 91 | MAZET J.-F. |